# EUROPEAN PATENT APPLICATION

(11) **EP 2 848 202 A1**
(43) Date of publication of application: **18.03.2015**
(21) Application number: 14183358.2
(22) Date of filing: 07.05.2012
(51) Int. Cl.: A61B 8/08

(54) **Echogenic sleeve**

(30) Priority: 06.05.2011 US 201161483098 P
(62) Divisional of application: 12722021.8
(71) Applicant: W.L. GORE & ASSOCIATES, INC., Newark, Delaware 19714-9206 (US)
(72) Inventor: Cully, Edward H, Flagstaff, AZ Arizona 86004 (US); Flury, Keith M, Flagstaff, AZ Arizona 86004 (US)
(74) Representative: Shanks, Andrew

(57) **Abstract**

Devices with enhanced visualization in ultrasound imaging are provided. An echogenically enhanced interventional device comprising: an interventional device to be imaged ultrasonically; and an echogenic polymeric sleeve with adjustable topography positioned adjacent to the interventional device.

## Description

### CROSS REFERENCE TO RELATED APPLICATIONS

This application claims priority to provisional application Serial No. 6.1/483,098, filed May 6, 2011.

### FIELD OF THE INVENTION

The present invention relates to devices with enhanced echogenicity for better visualization in ultrasound imaging and methods for enhancing echogenicity of a device.

### BACKGROUND OF THE INVENTION

Ultrasound technology has advantages over other imaging modalities. Along with the health advantage of reducing or eliminating exposure to x-rays (fluoroscopy), the equipment needed is small enough to move on a hand cart. It also has advantages in diagnosing sub-surface tissue morphology. Furthermore, ultrasound transducers can be made small enough to place inside the body where they can provide better resolution than is currently available with external ultrasound imaging, magnetic resonance imaging, and x-ray computed tomography. Further, instrument enhancements, which increase echogenicity under ultrasound imaging, enable clinicians to quickly and more properly treat patients, saving time and money.

Many interventional tools and instruments are designed with polished surfaces that render the instruments virtually invisible on ultrasound. Interventional tools and instruments are herein referred to as "device(s)". The present invention relates to an enhancement to increase echogenicity of interventional devices.

Ultrasound image device enhancement or "echogenicity" has been studied for many years. When sound waves contact a smooth surface, the angle of incidence and reflection are the same. If the object is located at a steep angle, most or all the sound waves bounce away from a transmitting/receiver source. With such steep angles, even highly reflective devices can be invisible by ultrasound if scattering does not direct sound back to a source transducer. Conversely, if an object is perpendicular, the sound waves reflecting directly back may cause a "white out" effect and prevent the operator from seeing around the object. This affect is referred to as specular reflection.

Medical device manufacturers have tried a variety of techniques to improve visibility of devices to ultrasound. Examples include roughening the surface of the device, entrapping gas, adhering particles to substrate surfaces, creating indentations or holes in the substrates, and using dissimilar materials.

### SUMMARY OF THE INVENTION

An aspect of the present invention relates to an echogenically enhanced interventional tool or instrument which comprises the interventional tool or instrument to be imaged ultrasonically and an echogenic polymeric sleeve with adjustable topography positioned adjacent to the interventional tool or instrument. In one embodiment, the echogenic polymeric sleeve surrounds at least a portion of the interventional tool or instrument. The polymeric sleeve may be affixed in at least one location to the interventional tool or instrument. An adjustment means is provided so the topography of this polymeric sleeve can be altered while the interventional tool or instrument is in use.

Another aspect of the present invention relates to a method for enhancing echogenicity of an interventional tool or instrument. In this method, an echogenic polymeric sleeve with adjustable topography is positioned adjacent to the interventional tool or instrument. In one embodiment, the echogenic polymeric sleeve is positioned so that it surrounds at least a portion of the interventional tool or instrument. This method provides an adjustment means so the topography of this polymeric sleeve can be altered while the interventional tool or instrument is in use.

In another embodiment, a polymeric sleeve is provided that can be slipped onto or around an interventional tool or instrument and subsequently secured to the interventional tool or instrument in at least one location. An adjustment means is provided so the topography of this polymeric sleeve can be altered while the interventional tool or instrument is in use.

### BRIEF DESCRIPTION OF THE FIGURES

Figure 1 shows an interventional tool or instrument with an echogenic polymeric sleeve with adjustable topography positioned adjacent to the instrument.
Figure 2 shows the same interventional tool or instrument wherein the topography of the polymeric sleeve has been adjusted by shortening the sleeve length with respect to the interventional tool or instrument, thereby increasing its echogenicity.
Figure 3 is a bar graph showing results of a comparison of the dB increase above control of an instrument of the present invention with a shortened polymeric sleeve as depicted in Figure 2 and another commercially available coated instrument.
Figure 4 is a plot of the reflected energy at various angles, which reflects increased echogenic response.

### DETAILED DESCRIPTION OF THE INVENTION

The echogenically enhanced interventional tool or device of the present invention comprises an interventional tool or device to be imaged ultrasonically and an echogenic polymeric sleeve with adjustable topography positioned adjacent to the interventional device.

Examples of interventional devices which can be enhanced visually in ultrasound imaging in accordance with the present invention include, but are not limited to, medical devices such as permanent implantable or temporary indwelling devices such as catheters, guide wires, stents and other accessories and tools, and surgical instruments and needles such as septal puncture needles. However, as will be understood by the skilled artisan upon reading this disclosure, the techniques described herein for visually enhancing an interventional device via ultrasound imaging are adaptable to many different fields and devices.

In one embodiment of the present invention, the interventional device may be undetectable on its own by ultrasound. For example, the interventional device may have a polished surface that under ultrasound imaging renders the device virtually invisible.

In another embodiment, the interventional device may be echogenic. In this embodiment of the present invention the echogenic response of the interventional device may be similar to or different from the echogenic response of the polymeric sleeve adjacent thereto.

Echogenicity of this interventional device is enhanced in accordance with the present invention by positioning an echogenic polymeric sleeve with an adjustable topography adjacent to the interventional device.

In one embodiment, the echogenic polymeric sleeve surrounds at least a portion of the interventional device. The extent to which the polymeric sleeve surrounds the interventional device depends in part on how it is secured to the device and orientation of the sleeve on the device relative to the ultrasound imaging source.

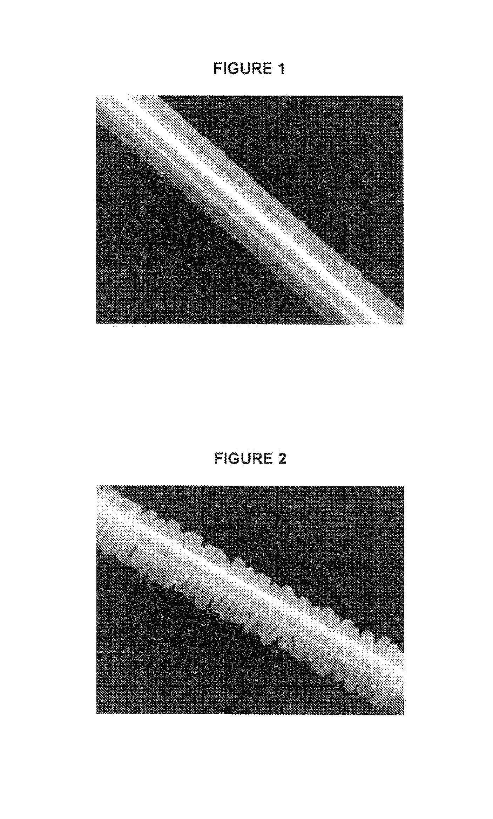

Echogenic response of the device of the present invention is initiated or modified actively by the user by adjusting the topography of the polymeric sleeve. In one embodiment, topography of the polymeric sleeve is adjusted by changing length of the polymeric sleeve with respect to the interventional device. For example, in one embodiment, as depicted in Figures 1 and 2, length of the echogenic polymeric sleeve is shortened with respect to the device thereby causing the sleeve to bunch up. This bunching results in corrugations, which cause an increase in echogenicity. In another embodiment, the length of a bunched-up echogenic polymer sleeve can be increased relative to the interventional device to reduce the topography and thereby decrease the echogenicity. Changes in topography of the polymeric sleeve of the present invention may be reversible or irreversible.

In an alternate embodiment, the topography of the sleeve may be adjusted by the introduction of a fluid to impart a change in echogenicity. In another embodiment, apparent density of the sleeve material may be adjusted, such as by the introduction of a fluid in a way that would impart a change in echogenicity; for example, if the material was porous and entrapped air was replaced by fluid, the echogenic response would change. Adjustment of echogenicity may also result from a change in thickness of the material. Said thickness change may be due to mechanical deformation, such as, but not limited to, twisting the material to apply a stress which reduces thickness, or pulling the material axially, whereby the material bunches up to increase thickness. Said compression may result from the rotation or wrapping of a compliant porous material around an axis of rotation. Compliant as used herein means any material capable of being compressed and/or expanded by an external force, such as PATT foam, silicone foam, and foamed fluoropolymers and fluoroelastomers.

Any biocompatible polymeric mesh or film capable of an echogenic response with minimal profile impact can be used. Examples of polymers useful in the sleeve of the present invention include, but are not limited to, expanded polytetrafluoroethylene (ePTFE), compliant polymeric foams, porous fluoropolymers, PET, polyurethane,Pebax and composites thereof. Commercially available polymeric meshes for use in the sleeve in the present invention include DUALMESH by Gore.

In one embodiment, the film comprises a very thin biocompatible membrane or film that can be formed into a tubular shape or wrapped onto an interventional device in a way that provides movement along the axis of the device. The material choice must be such that the corrugations in this material, when axially compressed (or allowed to axially compress) must become visible to ultrasound.

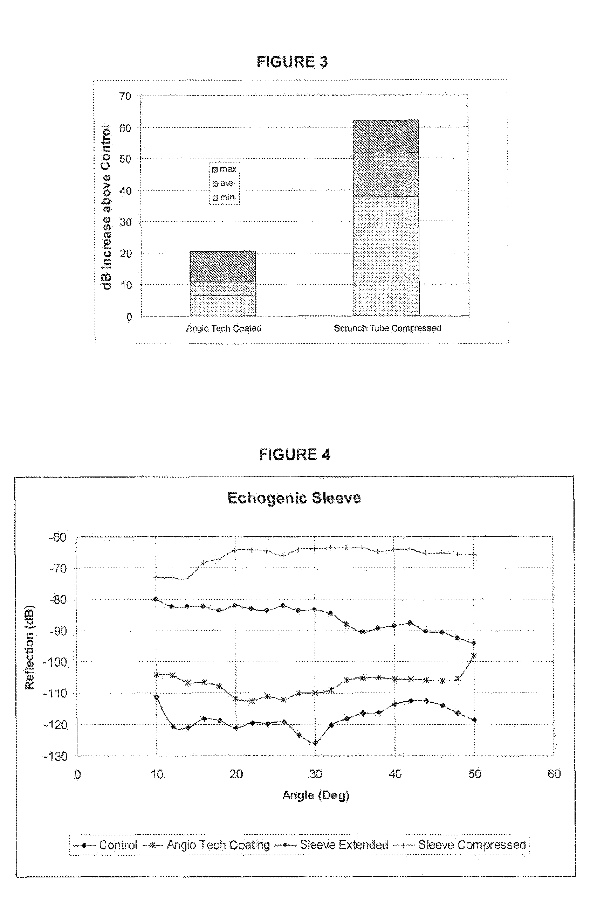

Enhanced echogenicity of a device of the present invention was demonstrated experimentally. Results are depicted in Figure 3 which shows a comparison of the dB increase of an embodiment of the invention above a control device and the dB increase above control of an Angiotech coated device

The following non-limiting examples are provided to further illustrate the present invention.

### EXAMPLES

### Example 1: Materials

A stainless steel needle with the dimensions of 0.040" diameter and approximately 4.8" long was used as the test device for echogenic enhancement. An unmodified needle was used as a control device to compare the results of the modification. Echogenicity of a stainless steel needle surrounded by a polymeric sleeve with adjustable topography in accordance with the present invention was also compared to an Angiotech coated needle (Angiotech Pharmaceuticals, Inc., 1618 Station Street Vancouver, BC Canada V6A 1 B6).

### Example 2: Methods

Three different methods were used to evaluate and compare the examples and control devices.

All samples were subjected to an acoustic wave imaging system. The testing apparatus consisted of a 7.5 MHz transmitting/receiving transducer mounted onto a flat bar with a sample holder placed approximately 2.5 cm at the transducer's focal length. The 7.5 MHz transducer produced a wave length (A) of 200 microns. At 2.5 cm the width of the signal was approximately 1 mm. The needle sample was placed into a holder that is perpendicular to the axis of the emitting transducer. This is 0 degrees. The sample holder is removable for ease of changing out the sample. The holder is magnetically held in a rotatable goniometer for measuring the angle of the sample relative to the transmitting and receiving transducer.

The sample and transducer were submerged into a room temperature water tank. Before collecting the data, every sample was aligned such that each was positioned the same distance from and oriented with the transducer. This was accomplished by increasing the attenuation setting on the pulser/receiver controller (approximately 40 dB) to prevent saturation of the received signal. The operator then visually monitored the wave signal while manually rotating the goniometer and dialing the fine adjustment knobs on the transducer to achieve a maximum return signal. The attenuation was adjusted to a reference point of approximately 1 volt. The attenuation setting and the goniometer indication were recorded. The goniometer was than rotated 10 degrees from the recorded indication. Since the signal typically decreases off of perpendicular (specular reading) the attenuation was reduced. The reduced level allowed a strong enough signal during collection, without saturation of the receiver. The sample was rotated through the entire angular rotation to ensure that the signal did not saturate or significantly move away from or closer to the transducer moving the signal out of the data collection window. Time shift was monitored. Significant time shift may indicate that the transducer was not aligned with the center or pivot of the sample. Once the set-up was completed, the goniometer was moved to the 10 degree mark and the collection of points was taken to 50 degrees at 2 degree increments. Equipment connected to the transducer and test fixture measured reflection. The Lab View software and hardware were used for data collection and later analysis.

A second evaluation of samples was performed in a silicone phantom submersible in a blood substitute from ATS laboratories to increase attenuation and create a more realistic image environment. Using a 6.5 mHz transducer ultrasound system, the samples were inserted into the phantom. A still image was captured for each sample. These images were visually compared to control images and inspected for consistency with the transducer 2D data. The data was collected at three different times. Between collections two and three the transducer was rebuilt. Thus, while the absolute dB scale of plots is not the same, the relative deltas are of importance.

A comparison of the dB increase above control of a device of the present invention and an Angiotech coated device is depicted in Figure 3.

## Claims

1. An echogenically enhanced interventional device comprising:
(a) an interventional device to be imaged ultrasonically; and
(b) an echogenic polymeric sleeve with adjustable topography positioned adjacent to the interventional device.

2. The echogenically enhanced interventional device of claim 1 wherein the echogenic polymeric sleeve surrounds at least a portion of the interventional device.

3. The echogenically enhanced interventional device of claim 1 wherein topography of the polymeric sleeve is adjusted by changing length of the polymeric sleeve with respect to the interventional device.

4. The echogenically enhanced interventional device of claim 3 wherein length of the echogenic polymeric sleeve is shortened.

5. The echogenically enhanced interventional device of claim 3 wherein length of the echogenic polymeric sleeve is lengthened.

6. The echogenically enhanced interventional device of claim 3 wherein adjustment by changing length of the polymeric sleeve with respect to the interventional device is reversible.

7. The echogenically enhanced interventional device of claim 3 wherein adjustment by changing length of the polymeric sleeve with respect to the interventional device is irreversible.

8. The echogenically enhanced interventional device of claim 1 wherein the interventional device is a surgical instrument.

9. The echogenically enhanced interventional device of claim 1 wherein the interventional device is a septal puncture needle.

10. The echogenically enhanced interventional device of claim 1 wherein the interventional device is not echogenic.

11. The echogenically enhanced interventional device of claim 1 wherein the interventional device is echogenic.

12. The echogenically enhanced interventional device of claim 11 wherein the interventional device has an echogenic response different from the polymeric sleeve.

13. The echogenically enhanced interventional device of claim 1 wherein the polymeric sleeve comprises expanded polytetrafluoroethylene (ePTFE).

14. A method for enhancing echogenicity of an interventional device, said method comprising positioning adjacent to the interventional device an echogenic polymeric sleeve with adjustable topography.

15. The method of claim 13 wherein the echogenic polymeric sleeve surrounds at least a portion of the interventional device.

16. An echogenically enhanced interventional device comprising:
(a) an interventional device to be imaged ultrasonically; and
(b) an echogenic polymeric sleeve with adjustable thickness positioned adjacent to the interventional device.
